# EUROPEAN PATENT SPECIFICATION

(11) **EP 2 023 754 B1**
(45) Date of publication and mention of the grant of the patent: **16.03.2011**
(21) Application number: 07728918.9
(22) Date of filing: 09.05.2007
(51) Int. Cl.: A41D 13/005, A42B 3/28

(54) **COOLING SYSTEM BY CONTACT**
KONTAKTKÜHLSYSTEM
SYSTÈME DE REFROIDISSEMENT PAR CONTACT

(30) Priority: 10.05.2006 ES 200601192
(43) Date of publication of application: 18.02.2009
(73) Proprietor: Criogenius, S.L., 08100 Mollet Del Vallès (ES)
(72) Inventor: SERRANO MOLINA, Jose Antonio, E-08396 Sant Cebria de Vallalta (ES)
(74) Representative: Ponti Sales, Adelaida
(86) International application number: PCT/EP2007/054466
(87) International publication number: WO 2007/128823

(56) References cited:
- WO-A-00/18225
- WO-A-2006/029762
- AU-B2- 516 011
- DE-A1- 19 619 152
- GB-A- 1 198 354
- GB-A- 2 325 625
- JP-A- 7 310 208
- JP-A- 8 210 746
- US-A- 2 850 885
- US-A- 4 377 075
- US-A- 4 962 761
- US-A1- 2003 101 503
- US-B1- 6 551 347
- US-B1- 6 763 671
- MAYINGER, F.; STRAUB, J.: "Arbeitsunterlagen zu Vorlesung und Übung Wärme- und Stoffübertragung" 1988, TU MÜNCHEN, LEHRSTUHL FÜR THERMODYNAMIK , MÜNCHEN , XP002443606 pages 33-36,41 -& MAYINGER, F.;STRAUB, L.: "Aufgabensammlung zur Übung Wärme- und Stoffübertragung" 1988, TU MÜNCHEN, LEHRSTUHL FÜR THERMODYNAMIK , MÜNCHEN , XP002443607 Aufgabe 28 -& ANONYMOUS: "Musterlösungen zu den Übungsaufgaben" 1988, FACHSCHAFT MASCHINENBAU , MÜNCHEN , XP002443608 pages 69,70

## Description

The present invention refers to a cooling system by contact.

### BACKGROUND OF THE INVENTION

There are known different cooling systems by contact of a body or object, as will be described hereinafter.

US 5,438,707 and US 4,738,119 disclose, respectively, cooling devices of a body comprising a garment which houses a tubing network provided with little openings or micropores through which pressurized gas flows, such as air or carbon dioxide. Said pressurized gas, once injected, expands quickly through said openings or micropores cooling itself and absorbing a great quantity of heat, producing a cooling effect to the wearer of the garment.

Said devices present the drawback that they include at said garment a very extended and complex tubing circuit through which the cooling substance flows, resulting in a not very flexible garment. Furthermore, the tubes cool the garment only when the pressurized gas flows through them, but they cannot store frigories (negative calories) during a long time. In the other hand, any torn of said garment can affect the circuit, making the cooling device useless, and it can also promote a leak of said coolant which, depending on the kind of substance used, can involve a serious danger for the user of the device.

Furthermore, another drawback is that said devices cannot be used with refrigerants at a very low temperature, because in these devices the coolant contacts directly the wearer of the garment.

US 5,787,505 discloses a device for cooling or heating a body comprising a garment with one or several pockets provided to insert a cooling or heating package. The cooling package comprises tight compartments containing water or other material which changes its state when absorbs heat, to cool therefore the wearer of said garment.

US 5,038,779 discloses a therapeutic garment for back pains comprising at least one pocket at the lumbar area provided to house a cooling package. The pocket is made in one piece of a material folded on the garment.

Both devices discloses in this two documents have the drawback that they are not suitable to provide cooling at a very low temperature, because the cooling packages are in direct contact with the wearer of the garment.

The application of the international patent WO 2005112675, of the same applicant than the present invention, discloses a device for cooling a body comprising a garment in contact with said body, liquid nitrogen as coolant to cool said garment and storing means of said coolant. The garment comprises a layer of conducting fabric with a thermal conductivity suitable to transmit by contact the heat to said coolant.

This device has the drawback that the wearer of the garment must wear one or more deposits of liquid nitrogen, which the involved risk if there is a leak at each of said deposits. On the other hand, the use of cryogenic deposits makes the device expensive.

GB 2325625 refers to a to a pocket-like shield formed from inner and outer members. A coiled tube or a manifold through which a fluid may be passed is secured to the inner surface of the outer member and a gelatinous substance may be positioned between the tube(s) and the inner member.

According to the above-mentioned document, it is provided a device wherein the garment is cooled through a controlled and regulated cooling fluid flow connected to a reservoir. The use of said reservoir makes the device have little autonomy.

### DESCRIPTION OF THE INVENTION

The objective of the cooling system by contact of the present invention is to solve the drawbacks known in the art, providing a number of advantages which will be described hereinafter.

The present invention comprises a heat absorbing device according to claim 1, its use and a system with such a heat absorbing device.

Therefore, the use of a cryogenic deposit forming part of the cooling system by contact is not necessary, as at the state of the art, and the security is enhanced because there is no risk of leaks and/or explosion when the pressure is increased, and the cost is considerably reduced.

Furthermore, the system provides a great autonomy because it can store a great quantity of frigories (negative calories), about 20 to 40 Kilofrigories (negative kilocalories).

As stated above, in the system of the present invention, the cooling fluid is charged by an external charging device offering a pressure enough to inject said cooling fluid to the system.

When it is necessary to charge cooling fluid, the system of the invention is connected at that moment to the external charging device, transferring by injection the cooling fluid to the elements of the cooling system by contact, therefore transferring frigories (negative calories) thanks to the configuration of the elements of the system itself.

Preferably, said radiator is a metallic radiator and, advantageously, said container carryier a body with a lid

The system comprises means for conducting and distributing said cooling fluid to said at least one device for absorbing heat.

Advantageously, said means for conducting and distributing the cooling fluid include an intake duct provided at an end with an intake nozzle provided with a valve to be connected intermittently to the external charging device.

Preferably, said intake duct is joined at its other end to a distribution element distributing the cooling fluid to each device for collecting and storing frigories through secondary ducts.

Preferably, the attachment between the intake nozzle and the external charging device is of bayonet kind.

Advantageously, the system comprises control means for the temperature transfer.

Preferably, said control means for the temperature transfer comprises an inflatable air chamber provided between the cold transmission surface and the body or object to be cooled.

Advantageously, the system comprises means for storing the water generated by the condensation process, using it to increase the existing frigories (negative colories).

Preferably, said means for storing condensation water comprises an absorbent fabric.

Alternatively, the system comprises waterproofing means preventing the condensation water to contact the body or object to be cooled, and means for collecting and evacuating said condensation water.

Optionally, the system is housed inside a thermally sealed garment to prevent condensation inside it.

### BRIEF DESCRIPTION OF THE DRAWINGS

To make the description of that disclosed previously easier some drawings are attached in which, diagrammatically and only as a non-limitative example, a practical case of embodiment of the cooling system by contact in shown, in which:
Fig. 1 is a frontal perspective view of a garment provided with the cooling system by contact, according to a first embodiment of the invention;
Fig. 2 is a rear perspective view of the garment of Fig. 1;
Fig. 3 is a perspective view of the radiator;
Fig. 4 is a perspective view of the radiator placed inside the container without a lid;

### DESCRIPTION OF A PREFERRED EMBODIMENT

Hereinafter a preferred embodiment of the cooling system by contact is described.

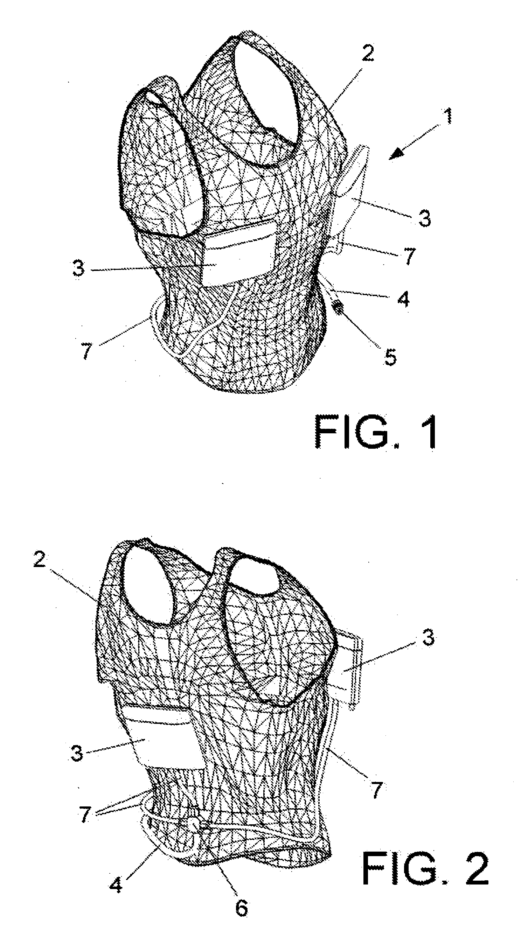

This embodiment is shown in Figs. 1 to 4, where it can be seen that the cooling system 1 by contact is applied to a garment 2, such as a vest.

In this case, the cooling system 1 by contact comprises three devices 3 for absorbing heat arranged on the vest 2, and that can receive a preset quantity of cooling fluid, specifically liquid nitrogen, from an external charging device (not shown).

To do this, the system 1 comprises means to conduct and distribute the liquid nitrogen comprising an intake duct 4 provided at an end with an intake nozzle 5 provided with a valve to be connected intermittently to the external charging device. Said intake duct 4 is connected by its other end to a distributing element 6 which distributes the liquid nitrogen to each device 3 for absorbing heat through secondary ducts 7.

The attachment between the intake nozzle 5 and the external charging device can be a system of bayonet kind, because it is an efficient and comfortable attachment. Therefore, it is assured that there are no disconnection problems from the contractions produced when the liquid nitrogen flows. The nozzle 5 can be housed inside the vest 2, and it can be uncovered by a zip, and when the charge is finished, it can be hidden inside the garment 2.

The design of the ducts 4 and 7 is ergonomic, so that they can remain disguised and they do not disturb the wearer.

The material suitable for the conduction and distribution means 4 to 7 of the liquid nitrogen is a non-metallic material such as Teflon, Pyrex, Armaflex, among others, the metallic material being not suitable because it can crack.

Another option consists in not using conducting and distributing means f the liquid nitrogen, so that each heat absorbing device 3 is fed directly from the external charging device. Therefore, the ergonomics of the garment 2 is enhanced.

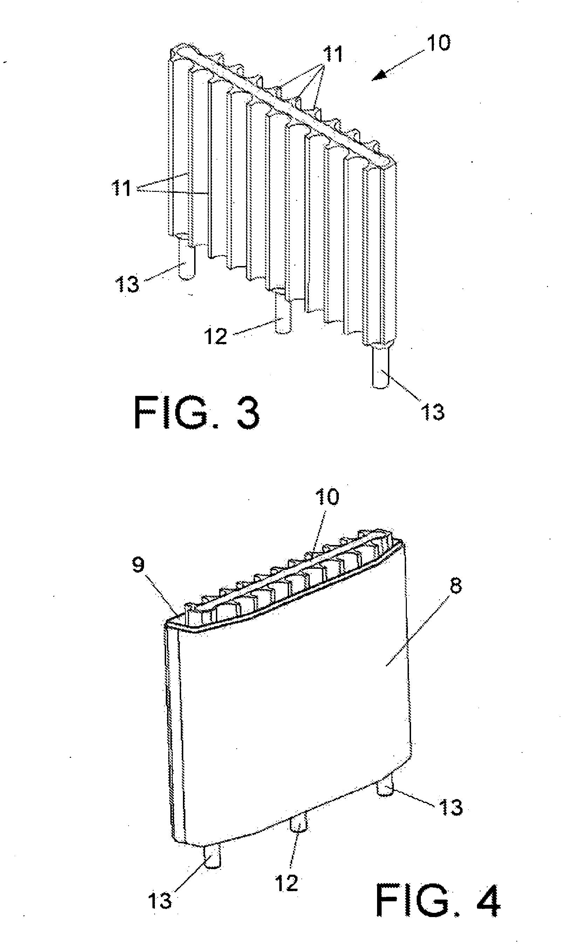

With reference to Figs. 3 and 4, each heat absorbing device 3 comprises a container 8 thermally isolated, except a wall 9 with a minimum width, which will contact the garment 2, acting as a cold transmission surface, as will be explained hereinafter.

The container 8 can be made from a plastic material, such as polyurethane; and the cold transmission surface 9 can be made from plastic o metallic material, taking into account that the metal will provide to the garment 2 a temperature lower than about -30°C, compared with the plastic material, which will provide about -15°C.

The container 8 comprises a body with a lid which houses inside it a radiator 10 provided with internal channels (not shown) and external wings 11. The material suitable for the radiator 10 is a metal, such as aluminum, copper, and steel, among others.

The charge of liquid nitrogen is injected inside the radiator 10 through an intake duct 12, and flows through internal channels, so that said radiator 10 can collect frigories (negative calories) from the nitrogen when it pass through the channels, and it can increase the temperature from - 198°C (temperature of the liquid nitrogen) to about -160°C (temperature at which the nitrogen changes its state from liquid to gas). The gas inside the radiator 10 exits outside the container 8 through exit ducts 13, providing the last frigories (negative calories) to the system 1.

On the other hand, the container 8 comprises a viscous gel (not shown) surrounding the metallic radiator 10 and filling the volume of the container 8 not filled by the radiator 10. The design of the radiator 10 is optimized thanks to the external wings 11 to provide a maximum contact area with the gel.

Said gel has a thermal conductivity suitable for storing transferred from the metallic radiator 10 by contact, passing from about -160°C to about -40°C.

It must be pointed out that the gel permits to transmit cold slowly during a preset time to the cold transmission surface 9 of the container 8, which receives a temperature of about -15°C. This temperature is suitable to be supported by the human body during about two hours when it is under conditions of environmental heat of more than 100°C, as is the case of firemen. The system can provide between 20 and 40 Kilofrigories (negative kilocalories), providing a great autonomy.

The used gel can be any known gel, whose thermal properties permit to reach a suitable final temperature and to transmit cold during a preset time. Even though gel is the more suitable material because of its easy placement and adaptation to the external surface of the metallic radiator 10, it must be pointed out that it could be used a plastic material in the place of gel, such as polypropylene, polyethylene, among others.

The container 8 makes, therefore, the functions of containing the radiator 10 and the gel, and also of permitting the transfer of temperature through the surface 9 presenting a thickness of 0.2 mm.

Therefore, in this embodiment there are three layers transmitting frigories (negative calories) between the liquid nitrogen and the garment 2, which are: the metallic radiator 10, the gel, and the surface 9 which transmits cold of the container.

The system 1, once assembled, can be auto-portable, i.e. it can comprise a fixation between the different components by elastic harnesses and straps.

In the described embodiment control means of the transfer of temperature can be used, consisting in an air chamber provided between the cold transmission surface and the human body, and said air chamber can be inflated when the final temperature is too low

It must be pointed out that in some environments, the container can present a water condensation greater than that expected. When the user pretends to use the own condensation, the system can include means to store the water generated by the condensation process, using it afterwards to increase the existing frigories (negative calories). To do this, said means for storing the condensing water can comprise an absorbent fabric.

When it is not wished to use the condensation water, the system can comprise waterproofing means preventing the condensation water to contact the body or object to be cooled, and means to collect and evacuate said condensation water. E.g. in the case of a garment it can include a tube at its lower part to evacuate the water.

Furthermore, the system of the invention can be housed inside a thermally sealed garment to prevent condensation inside it.

## Claims

1. A heat absorbing device (3) for a cooling system by contact to be engaged on a garment, said device being able to absorb heat coming from a body or object through a cold transmission surface (9) of a thermal transmission element in indirect contact with said body or object, **characterized in that** said device (3) comprises a thermally isolated container (8), with the exception of the cold transmission surface (9) intended for being in indirect contact with said body or object to be cooled, said container (8) comprising a radiator (10) provided with internal channels and external wings (11), said radiator being able to receive a preset quantity of liquid nitrogen from an external charging device, said container comprising ducts (13) to exit the gas inside the radiator (10) coming from the evaporation of said liquid nitrogen when in contact with the radiator (10), and **in that** said container (8) also comprises a viscous gel around the radiator (10), filling the volume of the container (8) not occupied by the radiator (10), said viscous gel being able to store the frigories transferred by contact from the radiator (10), and said thermal transmission element (9) presenting a thermal conductivity suitable to obtain a final preset temperature during an also preset time, on said cold transmission surface.

2. A device (3) according to claim 1, **characterized in that** said radiator (10) is a metallic radiator.

3. A device (3) according to claim 1, **characterized in that** said container (8) comprises a body with a lid.

4. Use of a device (3) according to claim 1, wherein said radiator (10) intermittently receives said liquid nitrogen from said external charging device, said liquid nitrogen changing its state from liquid to gas inside the radiator (10), said gas exiting outside the container (8) through said exit ducts (13), the frigories coming from the evaporation of the liquid nitrogen being transferred by contact of said radiator (10) to said viscous gel.

5. A cooling system by contact (1) to be engaged on a garment, comprising said liquid nitrogen to cool a body or object, and at least a heat-absorbing device (3) according to claims 1 to 3.

6. A system according to claim 5, **characterized in that** it comprises means (4, 5, 6, 7) for conducting and distributing said liquid nitrogen to said at least one device (3) for absorbing heat.

7. System (1) according to claim 6, **characterized in that** said means for conducting and distributing the liquid nitrogen include an intake duct (4) provided at an end with an intake nozzle (5) provided with a valve to be connected intermittently to the external charging device.

8. System (1) according to claim 7, **characterized in that** said intake duct (4) is joined at its other end to a distribution element (6) distributing the cooling fluid to each device (3) for absorbing heat through secondary ducts (7).

9. System (1) according to claims 7 or 8, **characterized in that** the attachment between the intake nozzle (5) and the external charging device is of bayonet kind.

10. System (1) according to anyone of the claims 5 to 9, **characterized in that** it comprises control means for the temperature transfer.

11. System (1) according to claim 10, **characterized in that** said control means for the temperature transfer comprises an inflatable air chamber provided between the cold transmission surface (9) and the body or object to be cooled.

12. System (1) according to anyone of the claims 5 to 11, **characterized in that** it comprises means for storing the water generated by the condensation process, using it to increase the existing frigories .

13. System (1) according to claim 12, **characterized in that** said means for storing condensation water comprises an absorbent fabric.

14. System (1) according to anyone of the claims 5 to 13, **characterized in that** it comprises waterproofing means preventing the condensation water to contact the body or object to be cooled, and means for collecting and evacuating said condensation water.

15. A garment comprising a system (1) according to anyone of the claims 5 to 14, **characterized in that** the system (1) is housed inside a thermally sealed garment to prevent condensation inside it.

## Patentansprüche

1. Wärmeabsorptionsvorrichtung (3) für ein Kontaktkühlsystem zum Eingriff auf bzw. Einsatz bei einem Kleidungsstück, wobei die Vorrichtung zum Absorbieren von Wärme fähig ist, welche von einem Körper oder Gegenstand durch eine Kälteübertragungsfläche (9) eines Wärmeübertragungselements kommt, welches in indirektem Kontakt mit dem Körper oder Gegenstand seht, **dadurch gekennzeichnet, dass** die Vorrichtung (3) mit Ausnahme der Kälteübertragungsfläche (9), welche vorgesehen ist, um mit dem zu kühlenden Körper oder Gegenstand in indirektem Kontakt zu stehen, einen thermisch isolierten Behälter (8) aufweist, wobei der Behälter (8) einen Kühler (10) aufweist, welcher mit Innenkanälen und externen Flügeln (11) versehen ist, der Kühler zum Aufnehmen einer vorbestimmten Menge an Flüssigstickstoff von einer externen Ladevorrichtung fähig ist und der Behälter Leitungen (13) zum Ausscheiden des Gases in den Kühler (10) aufweist, welches aus der Verdampfung des Flüssigstickstoffs bei Kontakt mit dem Kühler (10) stammt, und **dadurch, dass** der Behälter (8) auch ein zähflüssiges Gel um den Kühler (10) herum aufweist, welches das Volumen des Behälters (8) füllt, welches nicht durch den Kühler (10) eingenommen ist, wobei das zähflüssige Gel zum Speichern der durch den Kontakt vom Kühler (10) übertragenen Frigories fähig ist und das Wärmeübertragungselement (9) eine Wärmeleitfähigkeit darstellt, welche zum Erhalten einer vorbestimmten Endtemperatur während einer ebenso vorbestimmten Zeit auf der Kälteübertragungsfläche geeignet ist.

2. Vorrichtung (3) nach Anspruch 1, **dadurch gekennzeichnet, dass** der Kühler (10) ein Metallkühler ist.

3. Vorrichtung (3) nach Anspruch 1, **dadurch gekennzeichnet, dass** der Behälter (8) einen Körper mit einem Deckel aufweist.

4. Verwendung einer Vorrichtung (3) nach Anspruch 1, wobei der Kühler (10) den Flüssigstickstoff von der externen Ladevorrichtung intermittierend aufnimmt, der Flüssigstickstoff den Zustand desselben vom flüssigen Zustand zum gasförmigen Zustand im Kühler (10) ändert, das Gas den Behälter (8) durch die Austrittsleitungen (13) verlässt und die von der Verdampfung des Flüssigstickstoffs stammenden Frigories durch Kontakt des Kühlers (10) zum zähflüssigen Gel übertragen werden.

5. Kontaktkühlsystem (1) zum Eingriff auf einem Kleidungsstück, welches den Flüssigstickstoff zum Kühlen eines Körpers oder Gegenstands und zumindest eine Wärmeabsorptionsvorrichtung (3) nach den Ansprüchen 1 bis 3 aufweist.

6. System nach Anspruch 5, **dadurch gekennzeichnet, dass** dasselbe eine Einrichtung (4, 5, 6, 7) zum Leiten und Verteilen des Flüssigstickstoffs zu zumindest einer Vorrichtung (3) zum Absorbieren von Wärme aufweist.

7. System (1) nach Anspruch 6, **dadurch gekennzeichnet, dass** die Einrichtung zum Leiten und Verteilen des Flüssigstickstoffs eine Einlassleitung (4) enthält, welche an einem Ende mit einer mit einem Ventil versehenen Einlassdüse (5) versehen ist, um mit der externen Ladevorrichtung intermittierend verbunden zu werden.

8. System (1) nach Anspruch 7, **dadurch gekennzeichnet, dass** die Einlassleitung (4) am anderen Ende derselben mit einem Verteilerelement (6) verbunden ist, welches das Kühlfluid an alle Vorrichtungen (3) zum Absorbieren von Wärme durch Sekundärleitungen (7) verteilt.

9. System (1) nach Anspruch 7 oder 8, **dadurch gekennzeichnet, dass** der Anschluss zwischen der Einlassdüse (5) und der externen Ladevorrichtung ein Bajonett-Anschluss ist.

10. System (1) nach einem der Ansprüche 5 bis 9, **dadurch gekennzeichnet, dass** dasselbe eine Steuereinrichtung für die Temperaturübertragung aufweist.

11. System (1) nach Anspruch 10, **dadurch gekennzeichnet, dass** die Steuereinrichtung für die Temperaturübertragung eine aufblasbare Luftkammer aufweist, welche zwischen der Kälteübertragungsfläche (9) und dem zu kühlenden Körper oder Gegenstand vorgesehen ist.

12. System (1) nach einem der Ansprüche 5 bis 11, **dadurch gekennzeichnet, dass** dasselbe eine Einrichtung zum Speichern des durch das Kondensationsverfahren erzeugten Wassers aufweist, welche dasselbe zum Erhöhen der bestehenden Frigories verwendet.

13. System (1) nach Anspruch 12, **dadurch gekennzeichnet, dass** die Einrichtung zum Speichern des Kondenswassers ein Absorptionsgewebe aufweist.

14. System (1) nach einem der Ansprüche 5 bis 13, **dadurch gekennzeichnet, dass** dasselbe eine Wasserschutzeinrichtung, welche verhindert, dass das Kondenswasser den zu kühlenden Körper oder Gegenstand berührt, und eine Einrichtung zum Sammeln und Entleeren des Kondenswassers aufweist.

15. Kleidungsstück mit einem System (1) nach einem der Ansprüche 5 bis 14, **dadurch gekennzeichnet, dass** das System (1) in einem thermisch abgedichtetem Kleidungsstück untergebracht ist, um eine Kondensation in demselben zu verhindern.

## Revendications

1. Dispositif d'absorption de chaleur (3) pour un système de refroidissement par engagement d'un contact sur un vêtement, ledit dispositif étant capable d'absorber la chaleur provenant d'un corps ou d'un objet à travers une surface de transmission de froid (9) d'un élément de transmission thermique en contact indirect avec ledit corps ou objet, **caractérisé en ce que** ledit dispositif (3) comprend un réceptacle isolé thermiquement (8), à l'exception de la surface de transmission de froid (9) destinée à être en contact indirect avec ledit corps ou objet à refroidir, ledit réceptacle (8) comprenant un radiateur (10) muni de canaux internes et d'ailes externes (11), ledit radiateur étant à même de recevoir une quantité préétablie d'azote liquide depuis dispositif de chargement externe, ledit réceptacle comprenant des conduits (13) pour faire sortir le gaz à l'intérieur du radiateur (10) provenant de l'évaporation dudit azote liquide lorsqu'il est en contact avec le radiateur (10), et **en ce que** ledit réceptacle (8) comprend également un gel visqueux autour du radiateur (10), remplissant le volume du réceptacle (8) non occupé par le radiateur (10), ledit gel visqueux étant capable de stocker les frigories transférées par contact du radiateur (10), et ledit élément de transmission thermique (9) présentant une conductibilité thermique convenant pour obtenir une température préétablie finale pendant une période de temps également préétablie, sur ladite surface de transmission de froid.

2. Dispositif (3) selon la revendication 1, **caractérisé en ce que** ledit radiateur (10) est un radiateur métallique.

3. Dispositif (3) selon la revendication 1, **caractérisé en ce que** ledit réceptacle (8) comprend un corps avec un couvercle.

4. Utilisation d'un dispositif (3) selon la revendication 1, dans laquelle ledit radiateur (10) reçoit par intermittence ledit azote liquide dudit dispositif de chargement externe, ledit azote liquide passant de l'état liquide à l'état gazeux à l'intérieur du radiateur (10), ledit gaz sortant à l'extérieur du réceptacle (8) via lesdits conduits de sortie (13), les frigories provenant de l'évaporation de l'azote liquide étant transférées par contact dudit radiateur (10) audit gel visqueux.

5. Système de refroidissement par engagement d'un contact (1) sur un vêtement, comprenant ledit azote liquide pour refroidir un corps ou un objet et au moins un dispositif absorbant la chaleur (3) selon les revendications 1 à 3.

6. Système selon la revendication 5, **caractérisé en ce qu'**il comprend des moyens (4, 5, 6, 7) servant à conduire et distribuer ledit azote liquide audit au moins un dispositif (3) pour absorber la chaleur.

7. Système (1) selon la revendication 6, **caractérisé en ce que** lesdits moyens servant à conduire et distribuer l'azote liquide comprennent un conduit d'admission (4) équipé, à une extrémité, d'une buse d'admission (5) munie d'une soupape à raccorder par intermittence au dispositif de chargement externe.

8. Système (1) selon la revendication 7, **caractérisé en ce que** ledit conduit d'admission (4) est joint, à son autre extrémité, à un élément de distribution (6) distribuant le fluide de refroidissement à chaque dispositif (3) pour absorber la chaleur à travers des conduits secondaires (7).

9. Système (1) selon les revendications 7 ou 8, **caractérisé en ce que** la fixation entre la buse d'admission (5) et le dispositif de chargement externe est du type à baïonnette.

10. Système (1) selon l'une quelconque des revendications 5 à 9, **caractérisé en ce qu'**il comprend un moyen de commande pour le transfert de température.

11. Système (1) selon la revendication 10, **caractérisé en ce que** ledit moyen de commande pour le transfert de température comprend une chambre à air gonflable disposée entre la surface de transmission de froid (9) et le corps ou l'objet à refroidir.

12. Système (1) selon l'une quelconque des revendications 5 à 11, **caractérisé en ce qu'**il comprend un moyen pour stocker l'eau générée par le processus de condensation en l'utilisant pour augmenter les frigories existantes.

13. Système (1) selon la revendication 12, **caractérisé en ce que** ledit moyen pour le stockage de l'eau de condensation comprend un tissu absorbant.

14. Système (1) selon l'une quelconque des revendications 5 à 13, **caractérisé en ce qu'**il comprend un moyen résistant à l'eau empêchant l'eau de condensation de venir en contact avec le corps ou l'objet à refroidir et un moyen pour collecter et évacuer ladite eau de condensation.

15. Vêtement comprenant un système (1) selon l'une quelconque des revendications 5 à 14, **caractérisé en ce que** le système (1) est logé à l'intérieur d'un vêtement thermoscellé pour empêcher la condensation à l'intérieur de celui-ci.
